# EUROPEAN PATENT APPLICATION

(11) **EP 4 491 186 A1**
(43) Date of publication of application: **15.01.2025**
(21) Application number: 23767099.7
(22) Date of filing: 06.03.2023
(51) Int. Cl.: A61K 31/7048, A61P 9/10, A61P 3/06, A61P 9/12, A23L 33/125

(54) **SAIKOSAPONIN C AS COMPOSITION FOR TREATING OR PREVENTING AGING VASCULAR DISEASES INCLUDING ARTERIOSCLEROSIS**

(30) Priority: 07.03.2022 KR 20220028687; 03.03.2023 KR 20230028300
(71) Applicant: Agingtarget, Inc., Uiwang-si Gyeonggi-do 16006 (KR)
(72) Inventor: KIM, Byeong Mo, Seoul 06767 (KR)
(74) Representative: AWA Sweden AB
(86) International application number: PCT/KR2023/003013
(87) International publication number: WO 2023/171998

(57) **Abstract**

The present invention relates to Saikosaponin C as a modulator for vascular aging and arteriosclerosis resulting therefrom, and provides a composition comprising Saikosaponin C as an active ingredient for treating, preventing, or alleviating aging vascular diseases, diseases due to vascular aging, specifically, arteriosclerosis, or symptoms due to vascular aging. The composition of the present invention is expected to be of great use in the treatment, prevention, and alleviation of arteriosclerosis as a supplement, substitute, or combination of an existing arteriosclerosis treatment that lowers LDL-C.

## Description

### [Technical Field]

The present invention relates to Saikosaponin C as a composition for treating or preventing aging vascular diseases including arteriosclerosis.

### [Background Art]

The most fundamental functional changes of blood vessels associated with vascular aging are impaired vascular distensibility and arterial stiffness. In other words, the occurrence of arteriosclerosis is closely related to vascular aging.

However, current therapies for arteriosclerosis are mainly limited to statin drugs which lower low-density lipoprotein cholesterol (LDL-C) in the blood or stent surgery. Non-statin drugs are used in combination with or as supplements to statin drugs, and both statin and non-statin drugs lower LDL-C, which is bad cholesterol. These therapeutic agents for atherosclerosis have an advantage of having excellent therapeutic effects on arteriosclerosis by suppressing LDL-C production, but the therapeutic effects are temporary. When drug administration is ceased for about a week, LDL-C returns to original levels. Therefore, there is a limitation in that continuous drug administration is required.

Therefore, the need to develop a novel concept of arteriosclerosis treatment based on inhibition of vascular aging has arisen. In consideration of the fact that arteriosclerosis is a disease caused by vascular aging, the present invention is based on development of a therapeutic agent that can inhibit the onset of arteriosclerosis by maintaining the blood vessels healthy through inhibition of vascular aging. It was found that the therapeutic agent drastically reduces the number of administrations and can become a more fundamental therapeutic agent. Based thereon, the present invention has been completed. In addition, the therapeutic agent is based on inhibition of aging and thus is expected to have preventive effects as well as therapeutic effects, and thus to be more suitable for the treatment paradigm of age-related diseases such as arteriosclerosis.

### [Disclosure]

### [Technical Problem]

It was found that senescence was more prominent in the arteriosclerotic plaques than in other blood vessel areas, a number of the effective substances derived as vascular senescence inhibitors exhibited efficacy as therapeutic agents for atherosclerosis, and atherosclerosis is a disease caused by vascular senescence.

In addition, the present inventors found that Saikosaponin C inhibits vascular aging caused by vascular endothelial cell senescence, and inhibits monocyte-endothelial cell adhesion and macrophage foam cell formation by NLRP3 inflammasome, which play an important role in the early stage of atherosclerosis, and thus that Saikosaponin C may be used as a novel concept therapeutic agent for atherosclerosis treatment based on vascular senescence inhibition, and the present invention was completed.

### [Technical Solution]

Accordingly, it is one object of the present invention to provide a pharmaceutical composition for treating or preventing diseases caused by vascular senescence containing Saikosaponin C or a pharmaceutically acceptable salt thereof as an active ingredient.

It is another object of the present invention to provide a food composition for ameliorating or preventing diseases caused by vascular aging containing Saikosaponin C or a sitologically acceptable salt thereof as an active ingredient.

It is another object of the present invention to provide a pharmaceutical composition for treating or preventing symptoms caused by vascular senescence containing Saikosaponin C or a pharmaceutically acceptable salt thereof as an active ingredient.

It is another object of the present invention to provide a food composition for ameliorating or preventing symptoms caused by vascular aging containing Saikosaponin C or a sitologically acceptable salt thereof as an active ingredient.

It is another object of the present invention to provide the use of Saikosaponin C or a pharmaceutically acceptable salt thereof as an active ingredient for preparation of a medicine for treating or preventing diseases caused by vascular senescence.

It is another object of the present invention to provide the use of Saikosaponin C or a sitologically acceptable salt thereof as an active ingredient for preparation of a food for ameliorating or preventing diseases caused by vascular aging.

It is another object of the present invention to provide the use of Saikosaponin C or a pharmaceutically acceptable salt thereof as an active ingredient for preparation of a medicine for treating or preventing symptoms caused by vascular senescence.

It is another object of the present invention to provide the use of Saikosaponin C or a sitologically acceptable salt thereof as an active ingredient for preparation of a food for ameliorating or preventing symptoms caused by vascular aging.

It is another object of the present invention to provide a method of treating or preventing diseases or symptoms caused by vascular senescence including administering Saikosaponin C or a pharmaceutically acceptable salt thereof as an active ingredient to a subject.

### [Advantageous effects]

The present invention relates to the use of Saikosaponin C which exhibits therapeutic effects on vascular senescence and arteriosclerosis caused thereby, and provides a composition containing Saikosaponin C as an active ingredient for treating, preventing, and ameliorating diseases caused by vascular senescence or arteriosclerosis, and is effective in treating, preventing, and ameliorating vascular senescence and arteriosclerosis.

### [Description of Drawings]

FIG. 1 shows the result of MTT assay to determine whether or not Saikosaponin C alleviates the growth inhibition of vascular endothelial cells (HUVEC, HPAEC) caused by high concentration of glucose mimicking a high-fat diet condition.

FIG. 2 shows the result of Trypan blue staining to determine whether or not Saikosaponin C alleviates the growth inhibition of vascular endothelial cells (HUVEC, HPAEC) caused by high concentration of glucose.

FIG. 3 shows the results of SA-β-gal staining and measurement of the expression of vascular senescence marker proteins (PAI-1, ICAM-1, VCAM-1) to determine whether or not Saikosaponin C alleviates the senescence of vascular endothelial cells (HUVEC, HPAEC) caused by high concentration of glucose.

FIG. 4 shows the results of cell cycle analysis and measurement of the expression of G1 phase growth arrest-inducing proteins (p21, p27, p16) to determine whether or not Saikosaponin C alleviates the G1 phase growth arrest of vascular endothelial cells (HUVEC, HPAEC) caused by high concentration of glucose.

FIG. 5 shows the result of fluorescence analysis to determine whether or not Saikosaponin C alleviates the generation of reactive oxygen species in vascular endothelial cells (HUVEC, HPAEC) caused by high concentration of glucose.

FIG. 6 shows the result of determination of whether or not Saikosaponin C alleviates the decrease in nitric oxide concentration and the decreases in nitric oxide synthase (NOS) activity, protein expression, and phosphorylation in vascular endothelial cells (HUVEC, HPAEC) caused by high concentration of glucose.

FIG. 7 shows the result of determination of whether or not Saikosaponin C alleviates the decreases in Sirt1 activity, protein expression, and phosphorylation in vascular endothelial cells (HUVEC, HPAEC) caused by high concentration of glucose.

FIG. 8 shows the result of evaluation of the roles of NOS and Sirt1 protein in the anti-vascular senescence effect of Saikosaponin C and the relationship between these proteins after treating cells with a NOS inhibitor and a Sirt1 inhibitor, in order to evaluate the mechanism of action on which Saikosaponin C alleviates the aging of vascular endothelial cells (HUVECs) caused by high concentration of glucose corresponding to a high-fat diet.

FIG. 9 shows the result of adhesion assay of monocytes labeled with fluorescent material (Calcein AM) to vascular endothelial cells (HUVEC, HCAEC) to determine whether or not Saikosaponin C inhibits monocyte-endothelial cell adhesion caused by TNF-α, a pro-atherosclerotic factor.

FIG. 10 shows the results of wound healing assay and transwell cell migration assay to determine whether or not Saikosaponin C inhibits abnormal proliferation and migration of vascular smooth muscle cells (AoSMC, CASMC) induced by PDGF, which is involved in the progression of atherosclerosis.

FIG. 11 shows the result of Oil Red O staining to determine whether or not Saikosaponin C inhibits macrophage foam cell formation caused by oxidized LDL (ox-LDL), a typical finding of early-stage atherosclerosis.

FIG. 12 shows the results of IL-1β secretion and caspase-1 cleavage assays to determine whether or not Saikosaponin C inhibits the activity of NLRP3 inflammasome, a protein complex that promotes macrophage-derived foam cell formation.

### [Best Mode]

Hereinafter, the present invention will be described in more detail.

Meanwhile, descriptions and embodiments provided herein may also be applied to other descriptions and embodiments. That is, all combinations of various elements disclosed herein fall within the scope of the present invention. In addition, the following specific description should not be construed as limiting the scope of the present invention.

The term such as "comprising" used herein should be understood as an open-ended term that has the possibility of including another element or embodiment, unless specifically stated otherwise in the phrase or sentence containing the term.

Terms or words used in the present specification and claims should not be construed as limited to ordinary or dictionary terms, and the present disclosure should be construed with meanings and concepts that are consistent with the technical idea of the present disclosure based on the principle that the inventors may appropriately define concepts of the terms to appropriately describe their own disclosure in the best way.

In one aspect, the present invention is directed to a pharmaceutical composition for treating or preventing diseases caused by vascular senescence containing Saikosaponin C or a pharmaceutically acceptable salt thereof as an active ingredient.

As used herein, the term "Saikosaponin C" refers to triterpene saponin which is a main component of the medicinal plant called "Bupleuri Radix". Saikosaponin C has a molecular weight of 927.12 and is represented by Formula 1 of C₄₈H₇₈O₁₇.

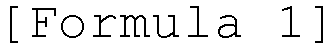

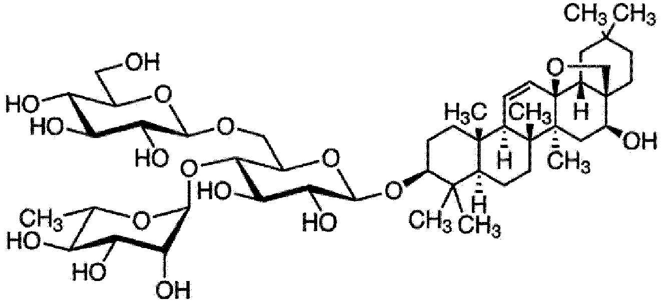

The pharmaceutical composition for treating or preventing diseases caused by vascular senescence containing Saikosaponin C or a pharmaceutically acceptable salt thereof according to the present invention has an effect of inhibiting vascular aging by inhibiting vascular endothelial cell senescence and may be useful as a novel approach to arteriosclerosis treatment based on vascular senescence inhibition by inhibiting monocyte-endothelial cell adhesion and macrophage-derived foam cell formation by the NLRP3 inflammasome, which play an important role in the early stages of arteriosclerosis.

As used herein, the term "disease caused by vascular aging" refers to a vascular disease caused by vascular endothelial cell senescence, including arteriosclerosis, and may be specifically arteriosclerosis caused by vascular endothelial cell senescence.

The composition containing Saikosaponin C or a pharmaceutically acceptable salt thereof according to the present invention inhibits monocyte-endothelial cell adhesion, NLRP3 inflammasome activity, macrophage-derived foam cell formation, or macrophage-derived foam cell formation by NLRP3 inflammasome, thereby treating or preventing arteriosclerosis, a disease caused by vascular senescence.

In the present invention, the pharmaceutical composition contains Saikosaponin C or a pharmaceutically acceptable salt thereof as an active ingredient, may further contain a pharmaceutically acceptable carrier, and may be prepared in the form of oral formulations such as powders, granules, tablets, capsules, suspensions, emulsions, syrups, aerosols, external preparations, and sterile injection solutions in accordance with conventional methods.

As used herein, the term "pharmaceutically acceptable salt" refers to any salt that has the desired biological and/or physiological activity of the compound and exhibits minimal undesirable toxicological effects. The pharmaceutically acceptable salt is prepared by an ordinary method known in the art and such a preparation method is known to those skilled in the art.

In the present invention, the pharmaceutically acceptable salt is a salt commonly used in the pharmaceutical industry and may, for example, include any one selected from the group consisting of: inorganic ion salts prepared from calcium, potassium, sodium, magnesium, and the like; inorganic acid salts prepared from hydrochloric acid, nitric acid, phosphoric acid, hydrobromic acid, iodic acid, perchloric acid, tartaric acid, sulfuric acid, and the like; organic acid salts prepared from acetic acid, trifluoroacetic acid, citric acid, maleic acid, succinic acid, oxalic acid, benzoic acid, tartaric acid, fumaric acid, mandelic acid, propionic acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid, and the like; sulfonic acid salts prepared from methanesulfonic acid, ethanesulfonic acid, benzenesulfonic acid, p-toluenesulfonic acid, naphthalenesulfonic acid, and the like; amino acid salts prepared from glycine, arginine, lysine, and the like; and amine salts prepared from trimethylamine, triethylamine, ammonia, pyridine, picoline, and the like, but is not limited thereto.

In the present invention, the pharmaceutical composition may contain a pharmaceutically acceptable carrier or additive. In the present invention, the term "pharmaceutically acceptable" means that a substance does not inhibit the activity of the active ingredient and does not have toxicity beyond that which the subject to which the substance is applied (prescribed) can adapt to. The term "carrier" is defined as a compound that facilitates the addition of the compound into cells or tissues.

Pharmaceutically acceptable carriers include, but are not limited to, those commonly used in the art, such as lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil.

In addition, the pharmaceutical composition of the present invention may contain a diluent or excipient such as a filler, a bulking agent, a binder, a wetting agent, a disintegrating agent, a surfactant, and other pharmaceutically acceptable additives.

The pharmaceutical composition of the present invention may be prepared in the form of a liquid, a suspension, a powder, a granule, a tablet, a capsule, a pill, or an extract.

The composition of the present invention may be administered orally or parenterally (e.g., by application of intravenous, subcutaneous, or intraperitoneal injection).

As used herein, the term "oral administration" refers to a method of injecting a drug to ameliorate a pathological symptom (periodontal disease) into the mouth, and as used herein, the term "parenteral administration" refers to a method of administering subcutaneously, intramuscularly, intravenously, or intraperitoneally using a tube, excluding oral administration.

Solid preparations for oral administration include powders, granules, tablets, capsules, soft capsules, and pills. Liquid preparations for oral administration include suspensions, liquids, emulsions, syrups, and aerosols. In addition to the commonly used simple diluents, such as water and liquid paraffin, liquid preparations for oral administration may contain various excipients, such as wetting agents, sweeteners, flavoring agents, and preservatives.

The preparations for parenteral administration may be obtained into external formulations such as sterilized aqueous solutions, liquids, non-aqueous solvents, suspensions, emulsions, eye drops, eye ointments, syrups, suppositories, aerosols, and the like, and sterilized injection preparations according to conventional methods, and preferably, pharmaceutical compositions may be prepared as creams, gels, patches, sprays, ointments, plasters, lotions, liniments, eye ointments, eye drops, patches, or cataplasms, but are not limited thereto. Compositions for topical administration may be anhydrous or aqueous depending on the clinical prescription. Non-aqueous solvents and suspensions may be used, such as propylene glycol, polyethylene glycol, vegetable oils such as olive oil, and injectable esters such as ethyl oleate. As a suppository base, Witepsol, macrogol, tween 61, cacao butter, laurin butter, glycerogelatin, or the like may be used.

A diluent, excipient or other pharmaceutically acceptable additive according to the present invention such as a filler, a bulking agent, a binder, a wetting agent, a disintegrating agent or a surfactant may be present in an amount of 0.1 to 99.9 wt%, specifically 0.1 to 50 wt% of the composition, but is not limited thereto.

The pharmaceutical composition for treating or preventing a disease caused by vascular senescence containing the Saikosaponin C or pharmaceutically acceptable salt thereof according to the present invention may be administered in a conventional manner via oral, rectal, intravenous, intraarterial, intraperitoneal, intramuscular, intrasternal, transdermal, topical, intraocular, or intradermal routes, and specifically may be administered orally.

The pharmaceutical composition of the present invention is administered in a pharmaceutically effective amount. The term "pharmaceutically effective amount" means an amount sufficient to treat a disease with a reasonable benefit/risk ratio applicable to medical treatment and the effective dosage may be determined according to factors including the type, severity, age, and gender of the subject, activity of the drug, sensitivity to the drug, administration time, administration route and excretion rate, treatment period, concurrently used drugs, and other factors well known in the pharmaceutical field. For example, when the pharmaceutical composition is administered once or several times a day, it may be administered at a dose of 0.001 mg/kg to 5 g/kg, and may be administered once a day or in several divided doses. In addition, the pharmaceutical composition of the present invention may contain 0.001 to 50 wt% of Saikosaponin C or a pharmaceutically acceptable salt thereof based on the total weight of the pharmaceutical composition.

The pharmaceutical composition of the present invention may further contain one or more active ingredients having the same or similar efficacy in addition to Saikosaponin C or pharmaceutically acceptable salt thereof, and may be administered in combination with a conventional vascular senescence inhibitor or a therapeutic agent for arteriosclerosis.

In another aspect, the present invention provides a food composition for ameliorating or preventing diseases caused by vascular aging containing Saikosaponin C or a sitologically acceptable salt thereof as an active ingredient.

As used herein, the term "Saikosaponin C" is described above.

As used herein, the term "disease due to vascular aging" is a vascular disease caused by aging of vascular endothelial cells, such as arteriosclerosis, and specifically, may be arteriosclerosis due to senescence of vascular endothelial cells.

The food composition of the present invention may contain an acceptable food supplement additive and may further contain an appropriate carrier, excipient, and diluent commonly used in the preparation of food.

The food composition of the present invention may include a formulation such as a pill, powder, granule, infusion, tablet, capsule, or liquid, and there is no particular limitation on the type of food to which the Saikosaponin C of the present invention or a sitologically acceptable salt thereof may be added. Examples of foods to which the substance may be added include: meat, sausage, bread, chocolate, candy, snacks, confectionery, pizza, ramen, other noodles, gum, dairy products including ice cream, various soups, beverages, tea, drinks, alcoholic beverages, and vitamin complexes.

In addition to Saikosaponin C or sitologically acceptable salt thereof, other ingredients may be added to the food composition and the types thereof are not particularly limited. For example, various herbal extracts, sitologically acceptable food additives, or natural carbohydrates may be contained as additional ingredients, as in ordinary foods, but are not limited thereto.

As used herein, the term "food additive" refers to an ingredient that may be added to food as an auxiliary, and may be appropriately selected from ingredients added to prepare each formulation of food by those skilled in the art. Examples of the food additive include various nutrients, vitamins, minerals (electrolytes), flavoring agents such as synthetic flavoring agents and natural flavoring agents, coloring agents, fillers, pectic acid and salts thereof, alginic acid and salts thereof, organic acids, protective colloid thickeners, pH regulators, stabilizers, preservatives, glycerin, alcohol, carbonating agents used in carbonated beverages, and the like, but the types of food additives of the present invention are not limited by the above examples.

Examples of the natural carbohydrates include: monosaccharides such as glucose and fructose; disaccharides such as maltose and sucrose; and polysaccharides such as dextrin, cyclodextrin, and sugar alcohols such as xylitol, sorbitol, and erythritol. In addition, other examples of flavoring agents include natural flavoring agents (such as thaumatin), stevia extracts (such as rebaudioside A, glycyrrhizin), and synthetic flavoring agents (such as saccharin, aspartame).

The food composition of the present invention may include a health functional food. In the present invention, the term "health functional food" refers to a food manufactured and processed in the form of tablets, capsules, powders, granules, liquids, and pills using raw materials or ingredients having useful functionality for the human body. Functionality means obtaining a useful effect for health maintenance such as regulating nutrients or physiological effects for the structure and function of the human body. The health functional food of the present invention may be prepared by a method commonly used in the art and may be prepared by adding raw materials and ingredients commonly added in the art during the preparation process. In addition, unlike general medicines, the health functional food is prepared from food and thus is free of side effects that may occur with long-term use of medicines, and is highly portable.

The amount of the active ingredient mixed may be appropriately determined depending on the purpose of use (prevention, health or therapeutic treatment). Generally, when a food is prepared, the active ingredient of the present invention may be added in an amount of 0.01 to 50 wt%, preferably 0.1 to 10 wt%, of the raw material composition, but is not limited thereto. However, in the case of long-term intake for the purpose of health and hygiene or for the purpose of health management, the amount may be used below the above range.

In another aspect, the present invention is directed to a pharmaceutical composition for treating or preventing symptoms caused by vascular senescence containing Saikosaponin C or a pharmaceutically acceptable salt thereof as an active ingredient.

As used herein, the terms "Saikosaponin C", "pharmaceutically acceptable salt", and "pharmaceutical composition" are as described above

The symptom caused by the vascular aging may be arteriosclerosis.

In addition, the symptom caused by the vascular aging or the symptom caused by the arteriosclerosis may include at least one selected from the group consisting of inhibition of vascular endothelial cell growth, increased vascular endothelial cell senescence, increased production of reactive oxygen species in vascular endothelial cells, decreased nitric oxide concentration in vascular endothelial cells, decreased expression or activity of NOS protein, decreased expression or activity of Sirt1 protein, increased monocyte-endothelial cell adhesion, increased NLRP3 inflammasome activity, increased macrophage-derived foam cell formation, and increased macrophage-derived foam cell formation by NLRP3 inflammasome.

The pharmaceutical composition containing the Saikosaponin C or a pharmaceutically acceptable salt thereof as an active ingredient according to the present invention has the effect of treating or preventing symptoms caused by vascular senescence as described above.

In another aspect, the present invention is directed to a food composition for ameliorating or preventing symptoms caused by vascular aging containing Saikosaponin C or a sitologically acceptable salt thereof as an active ingredient.

As used herein, the terms "Saikosaponin C", " sitologically acceptable salt", and "food composition" are as described above

The symptom caused by the vascular aging may be arteriosclerosis.

In addition, the symptom caused by the vascular aging or the symptom caused by the arteriosclerosis may include at least one selected from the group consisting of inhibition of vascular endothelial cell growth, increased vascular endothelial cell aging, increased production of reactive oxygen species in vascular endothelial cells, decreased nitric oxide concentration in vascular endothelial cells, decreased expression or activity of NOS protein, decreased expression or activity of Sirt1 protein, increased monocyte-endothelial cell adhesion, increased NLRP3 inflammasome activity, increased macrophage-derived foam cell formation, and increased macrophage-derived foam cell formation by NLRP3 inflammasome.

The food composition for ameliorating or preventing symptoms caused by vascular aging containing Saikosaponin C or a sitologically acceptable salt thereof as an active ingredient according to the present invention has the effect of ameliorating or preventing symptoms caused by vascular aging as described above.

In another aspect, the present invention is directed to the use of Saikosaponin C or pharmaceutically acceptable salt thereof as an active ingredient for preparation of a medicine for treating or preventing diseases caused by vascular senescence.

In another aspect, the present invention is directed to the use of Saikosaponin C or a sitologically acceptable salt thereof as an active ingredient for preparation of a food for ameliorating or preventing diseases caused by vascular aging.

In another aspect, the present invention is directed to the use of Saikosaponin C or a pharmaceutically acceptable salt thereof as an active ingredient for preparation of a medicine for treating or preventing symptoms caused by vascular senescence.

In another aspect, the present invention is directed to the use of Saikosaponin C or a sitologically acceptable salt thereof as an active ingredient for preparation of a food for ameliorating or preventing symptoms caused by vascular aging.

In another aspect, the present invention is directed to a method of treating or preventing diseases caused by vascular senescence or symptoms caused by vascular senescence including administering Saikosaponin C or a pharmaceutically acceptable salt thereof as an active ingredient to a subject.

As used herein, the terms "Saikosaponin C", "pharmaceutically acceptable salt", "sitologically acceptable salt", "diseases caused by vascular aging", "symptoms caused by vascular aging" and "food composition" are as described above.

As used herein, the term "subject" refers to all animals, including humans, that have developed diseases caused by vascular aging or symptoms caused by vascular aging. The subject includes mammals, birds, or the like, including cows, pigs, sheep, chickens, dogs, humans, or the like. The subject may be any subject in which the disease caused by vascular senescence or the symptom caused by vascular senescence is suppressed by the Saikosaponin C or pharmaceutically acceptable salt thereof according to the present invention, and in which the disease caused by vascular senescence or the symptom caused by vascular senescence is treated, without limitation.

### [Best Mode for Invention]

Hereinafter, embodiments of the present invention will be described in detail with reference to the accompanying drawings so that the present invention can be implemented by those skilled in the art. However, the present invention may be embodied in many different forms and is not limited to the embodiments described herein.

### Example 1. Preparation of Saikosaponin C

Saikosaponin C (SSc) is triterpene saponin which is a main component of the medicinal plant *"Bupleuri Radix".* Saikosaponin C has a molecular weight of 927.12 and is represented by formula of C₄₈H₇₈O₁₇. Saikosaponin C was used in the following experiments at concentrations of 5 µM and 20 µM and was purchased from Abcam (Cambridge, MA, USA).

### [Experimental Example]

### Experimental Example 1. Analysis of effects of Saikosaponin C on high glucose-induced inhibition of endothelial cell growth (metabolism analysis-MTT assay)

A high-fat diet test system at the cellular level was established using 33 mM high glucose (HG), which is 6 times the standard glucose concentration of 5.5 mM in the vascular endothelial cell culture medium.

Specifically, two types of endothelial cells, namely, human umbilical vein endothelial cells (HUVECs) and human pulmonary artery endothelial cells (HPAECs), were pretreated with 5 µM and 20 µM of Saikosaponin C (SSc) for 2 hours, and then treated with 33 mM of high glucose for 72 hours. Then, cell growth was analyzed using the MTT assay, which measures the mitochondrial metabolic capacity of living cells.

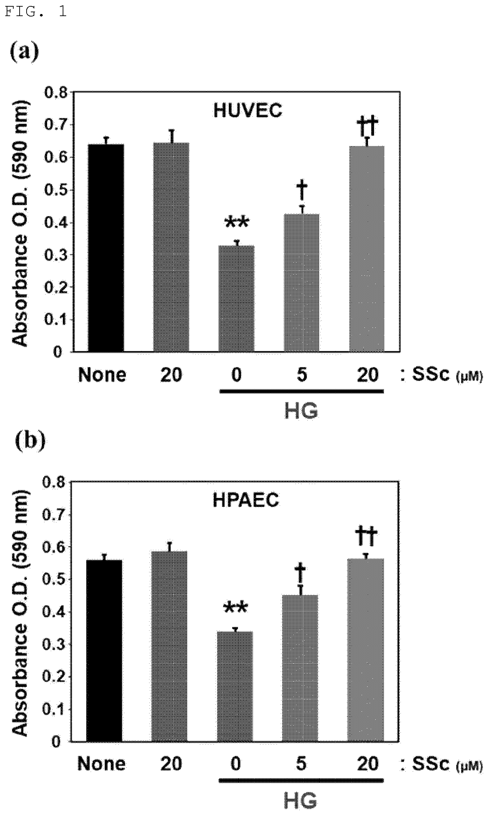

FIG. 1 shows the result of MTT assay to determine whether or not Saikosaponin C alleviates the growth inhibition of vascular endothelial cells (HUVEC, HPAEC) caused by high glucose as a high-fat diet. Endothelial cell growth was assessed by measuring the absorbance of MTT formazan at a wavelength of 590 nm.

As a result, the growth of HUVEC and HPAEC cells was inhibited by treatment with high glucose. However, pretreatment with Saikosaponin C significantly alleviated high glucose-induced cell growth inhibition in HUVEC and HPAEC in a dose-dependent manner.

### Experimental Example 2. Analysis of effects of Saikosaponin C on inhibition of vascular endothelial cell growth induced by high glucose (cell count analysis-Trypan blue staining)

Similar to Experimental Example 1, a high-fat diet evaluation system at the cell level was established.

Specifically, two types of vascular endothelial cells, namely, HUVEC and HPAEC cells, were pretreated with Saikosaponin C at concentrations of 5 µM and 20 µM for 2 hours, and then treated with high glucose at a concentration of 33 mM for 1 week. The number of viable cells was then analyzed by Trypan blue staining. The number of viable cells was based on the number of cells in the control group (none) set to "100".

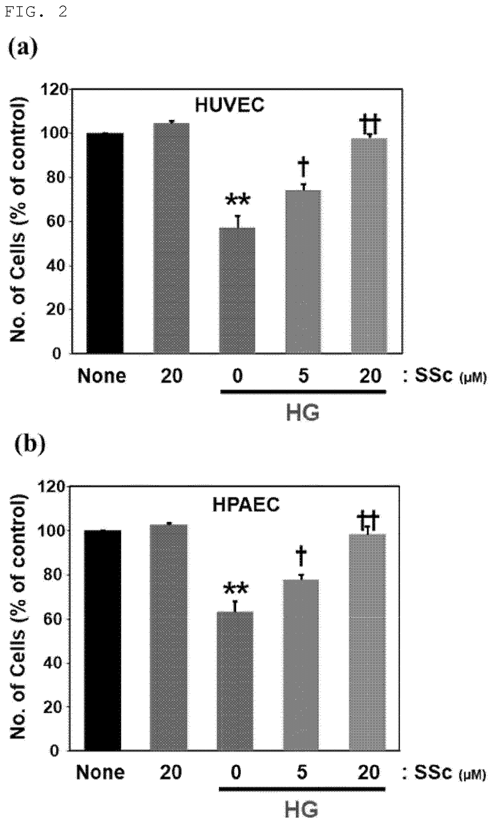

FIG. 2 shows the result of Trypan blue staining to determine whether or not Saikosaponin C alleviates the growth inhibition of vascular endothelial cells (HUVEC, HPAEC) caused by high glucose as a high-fat diet.

As a result, the growth of HUVEC and HPAEC cells was inhibited by high glucose treatment. However, pretreatment with Saikosaponin C significantly alleviated high glucose-induced cell growth inhibition in HUVEC and HPAEC in a dose-dependent manner.

### Experimental Example 3. Analysis of effects of Saikosaponin C on high glucose-induced vascular endothelial cell senescence (SA-β-gal staining and the analysis of vascular senescence marker protein expression)

Similar to Experimental Example 1, a high-fat diet evaluation system at the cellular level was established.

Specifically, two types of vascular endothelial cells, namely, HUVEC and HPAEC cells, were pretreated with Saikosaponin C at concentrations of 5 µM and 20 µM for 2 hours, and then treated with high glucose at a concentration of 33 mM for 72 hours (for SA-β-gal staining) or 48 hours (for vascular senescence marker protein analysis). Then, the degree of cellular senescence was measured through SA-β-gal staining and measured as the ratio of the total cells to cells stained in blue (SA-β-gal positive). In addition, the expression of vascular senescence marker proteins such as PAI-1, ICAM-1, and VCAM-1 was analyzed.

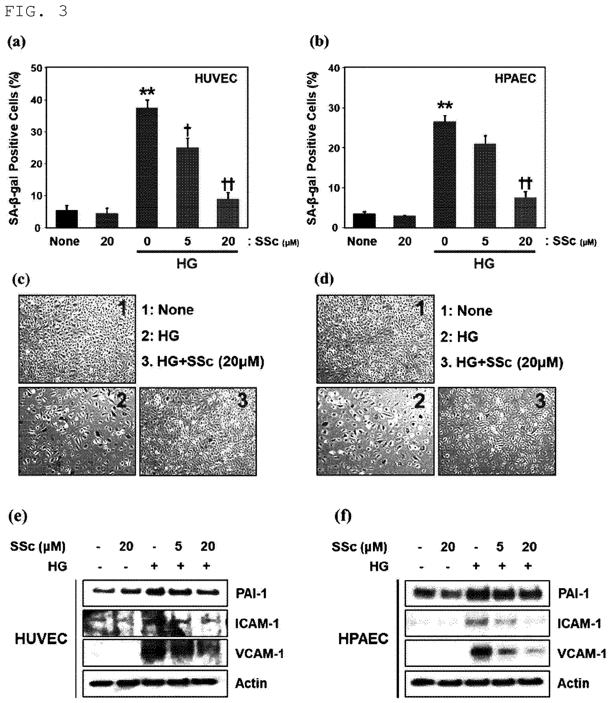

FIG. 3 shows the results of SA-β-gal staining and Western blotting to determine whether or not Saikosaponin C alleviates the senescence of vascular endothelial cells (HUVEC, HPAEC) caused by high glucose as a high-fat diet. Specifically, the senescence of vascular endothelial cells was evaluated by SA-β-gal staining to measure the activity of β-galactosidase, which is an enzyme present in the lysosome, an organelle within the cell (FIG. 3C and FIG. 3D) and this was represented as a graph (FIG. 3A and FIG. 3B). In addition, FIGS. 3E and 3F show the results of Western blotting to determine the expression of vascular senescence marker proteins such as PAI-1, ICAM-1, and VCAM-1 in two types of vascular endothelial cells (HUVEC, HPAEC).

As a result, the proportion of cells stained in blue (SA-β-gal positive) (FIG. 3A, FIG. 3B, FIG. 3C, and FIG. 3D) in HUVEC and HPAEC cells increased by high glucose treatment and the expression of vascular senescence marker proteins (FIG. 3E and FIG. 3F), which means that vascular cell senescence was induced by high glucose. However, pretreatment with Saikosaponin C significantly reduced high glucose-induced SA-β-gal positive cells and reduced the expression of vascular senescence marker proteins such as PAI-1, ICAM-1, and VCAM-1 in HUVEC and HPAEC in a dose-dependent manner.

### Experimental Example 4. Analysis of effects of Saikosaponin C on high glucose-induced G1 phase arrest (cell cycle analysis and the analysis of cell cycle regulatory protein expression)

Similar to Experimental Example 1, a high-fat diet evaluation system at the cellular level was established.

Specifically, two types of vascular endothelial cells, namely, HUVEC and HPAEC cells, were pretreated with Saikosaponin C at concentrations of 5 µM and 20 µM for 2 hours, then treated with high glucose (33 mM) for 48 hours, and then immobilized with cold 70% ethanol. The DNA of the immobilized vascular endothelial cells was stained with PI (propidium iodide, 50 ug/ml) solution and the cell cycle was analyzed using a flow cytometer. In addition, the expression of cell cycle regulatory proteins such as p21, p27, and p16 was analyzed after treatment with high glucose (33 mM) for 36 hours.

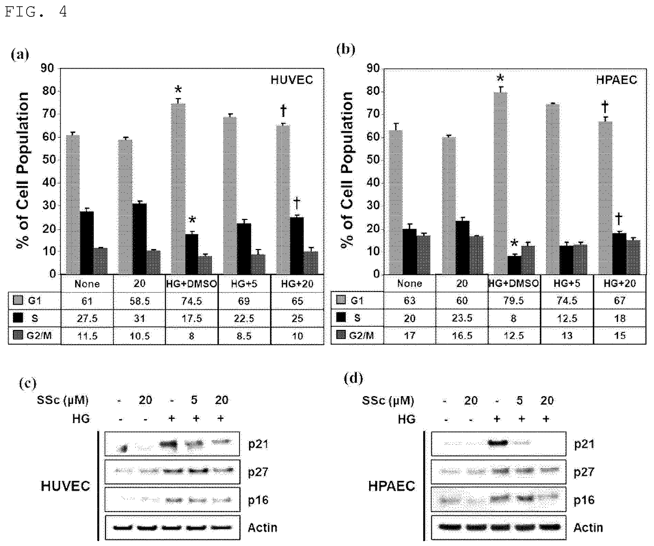

FIG. 4 shows the results of cell cycle analysis and measurement of the expression of G1 phase growth arrest-inducing proteins (p21, p27, and p16) to determine whether or not Saikosaponin C alleviates the G1 phase growth arrest of vascular endothelial cells (HUVEC, HPAEC) caused by high glucose as a high-fat diet. Since cellular senescence is closely related to the arrest in the G1 phase of the cell cycle, the cell cycle was evaluated using flow cytometry (FIG. 4A and FIG. 4B). In addition, the expression of p21, p27, and p16 proteins, which are G1 phase growth arrest-inducing proteins, was measured through Western blotting (FIG. 4C, FIG. 4D).

The result showed that the G1 phase arrest of the cell cycle and the increase in the expression of p21, p27, and p16 proteins were observed in HUVEC and HPAEC cells treated with high glucose. However, pretreatment with Saikosaponin C significantly abolished high glucose-induced G1 phase arrest and the increase in the expression of p21, p27, and p16 proteins in HUVEC and HPAEC in a dose-dependent manner. Since the G1 phase growth arrest is observed in the process of cell senescence, these results support the anti-vascular aging effect of Saikosaponin C.

### Experimental Example 5. Analysis of effects of Saikosaponin C on high glucose-induced generation of reactive oxygen species (ROS) (fluorescence image analysis)

Similar to Experimental Example 1, a high-fat diet evaluation system at the cellular level was established.

Specifically, two types of vascular endothelial cells, namely, HUVEC and HPAEC cells, were pretreated with Saikosaponin C at concentrations of 5 µM and 20 µM for 2 hours, and then treated with high glucose at a concentration of 33 mM for 24 hours. Then, hydrogen peroxide (H₂O₂) and superoxide anion (O₂^{·-}) generated in the cytoplasm and mitochondria were measured using each fluorescent probe. In addition, samples treated with 100 µM hydrogen peroxide (a ROS-inducing agent) for 1 hour and then treated for an additional 23 hours after medium change were used as positive control groups.

Different analytical reagents were used depending on the type of ROS and the intracellular location where they were generated. Specifically, cytosolic hydrogen peroxide, cytosolic superoxide anion, mitochondrial hydrogen peroxide, and mitochondrial superoxide anion were measured using H2DCFDA (2,7-dichlorodihydrofluorescein diacetate), DHE (dihydroethidium), DHR (dihydrorhodamine 123), and Mito-SOX reagents, respectively. The H2DCFDA (1 µM, 45 min staining), DHE (10 µM, 60 min staining), DHR (20 µM, 30 min staining), and Mito-SOX (5 µM, 10 min staining) were used as ROS-specific fluorescent probes for fluorescence image analysis.

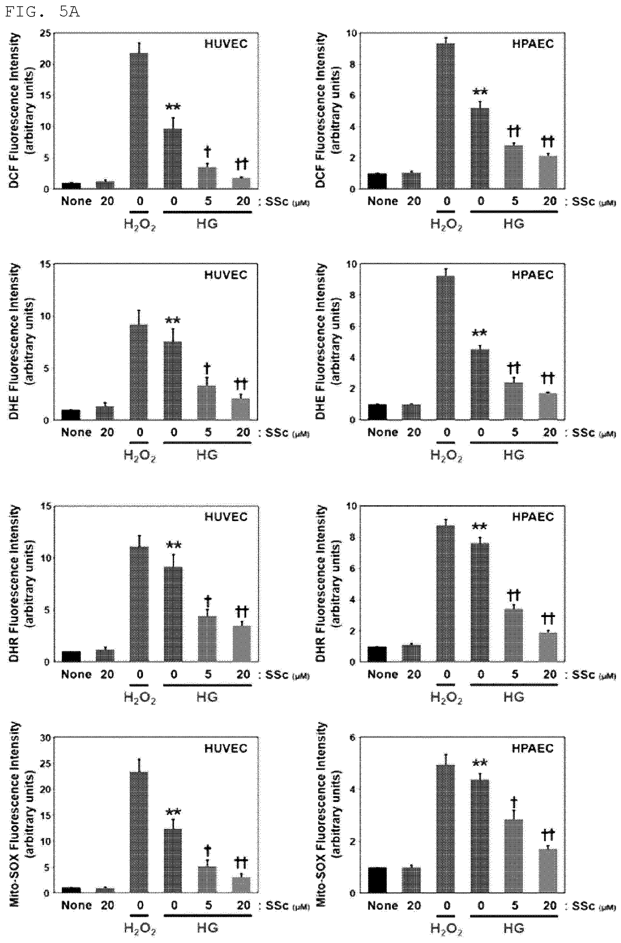

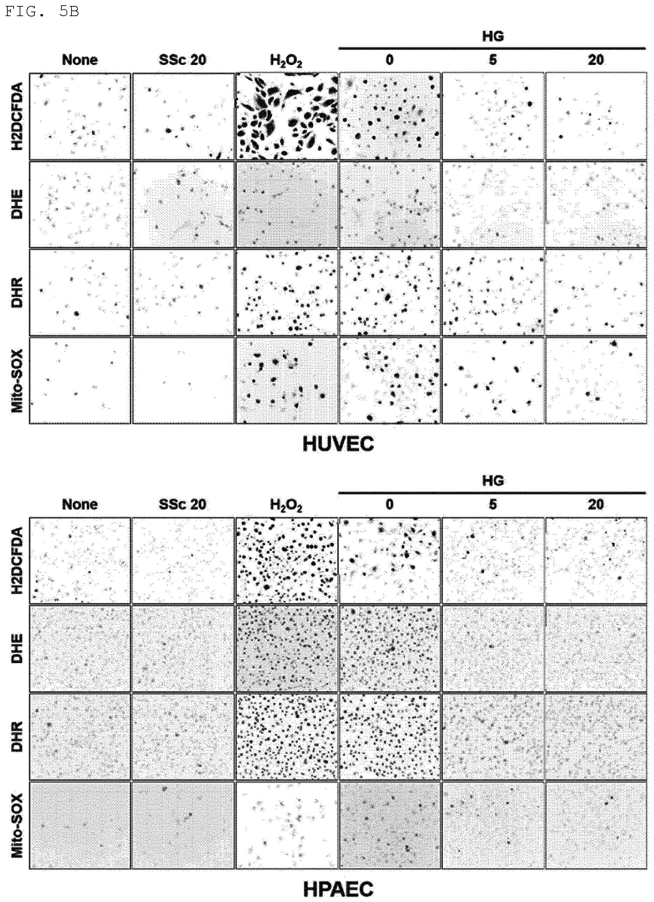

FIG. 5 shows the results of fluorescence analysis to determine whether or not Saikosaponin C alleviates the generation of ROS in vascular endothelial cells (HUVEC, HPAEC) caused by high glucose as a high-fat diet. FIG. 5B shows the result of fluorescence image analysis and FIG. 5A is a graph showing the fluorescence image analysis.

The result showed that the production of various ROS, such as intracytoplasmic hydrogen peroxide, intracytoplasmic superoxide anion, mitochondrial hydrogen peroxide, and mitochondrial superoxide anion, increased in HUVEC and HPAEC cells treated with high glucose. However, pretreatment with Saikosaponin C significantly suppressed high glucose-induced ROS generation in HUVEC and HPAEC in a dose-dependent manner. Hydrogen peroxide and superoxide anion are representative ROS. Therefore, these results support ROS-scavenging activity of Saikosaponin C.

### Experimental Example 6. Analysis of effects of Saikosaponin C on high glucose-induced reduction of NOS-NO signaling (analysis of nitric oxide concentration, nitric oxide synthase activity, and protein expression)

Similar to Experimental Example 1, a high-fat diet evaluation system at the cellular level was established.

Specifically, two types of vascular endothelial cells, namely, HUVEC and HPAEC cells, were pretreated with Saikosaponin C at concentrations of 5 µM and 20 µM for 2 hours, and then treated with high glucose at a concentration of 33 mM for 36 hours. Then, the nitric oxide (NO) concentration, endothelial nitric oxide synthase (eNOS) activity, protein expression, and phosphorylation level were analyzed.

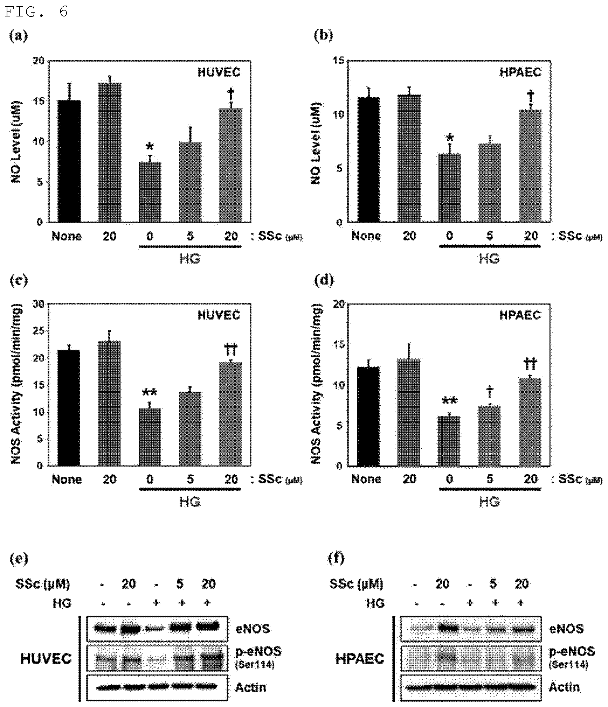

FIG. 6 shows the result of determination of whether or not Saikosaponin C alleviates NOS-NO signaling reduction in vascular endothelial cells (HUVEC, HPAEC) caused by high glucose as a high-fat diet. Nitric oxide production was measured using a Nitric Oxide Assay Kit (FIG. 6A and FIG. 6B). Specifically, nitrate (NO₃⁻) and nitrite (NO₂⁻) levels were measured using nitrate reductase and Griess reagent. Nitric oxide synthase activity was measured using a NOS Activity Assay Kit (FIG. 6C and FIG. 6D) and the expression and phosphorylation (Ser114) of NOS were measured by Western blotting (FIG. 6E and FIG. 6F).

The result showed that the nitric oxide (NO) level, the enzymatic activity, protein expression, and phosphorylation of Ser114 residue (p-eNOS) of eNOS were decreased by treatment of HUVEC and HPAEC cells with high glucose. However, pretreatment with Saikosaponin C significantly alleviated high glucose-induced decrease in NOS-NO signaling in HUVEC and HPAEC in a dose-dependent manner. NO produced by NOS is a vasodilator that induces vasorelaxation, contrary to reactive oxygen species, and helps keep blood vessels healthy. Therefore, these results support the anti-vascular aging effect of Saikosaponin C.

### Experimental Example 7. Analysis of effects of Saikosaponin C on high glucose-induced reduction of Sirt1 activity/expression (Sirt1 enzyme activity, protein expression, and phosphorylation analysis)

Similar to Experimental Example 1, a high-fat diet evaluation system at the cellular level was established.

Specifically, two types of vascular endothelial cells, namely, HUVEC and HPAEC cells, were pretreated with Saikosaponin C at concentrations of 5 µM and 20 µM for 2 hours, and then treated with high glucose at a concentration of 33 mM for 36 hours. Then, Sirt1 activity, protein expression and phosphorylation levels were analyzed.

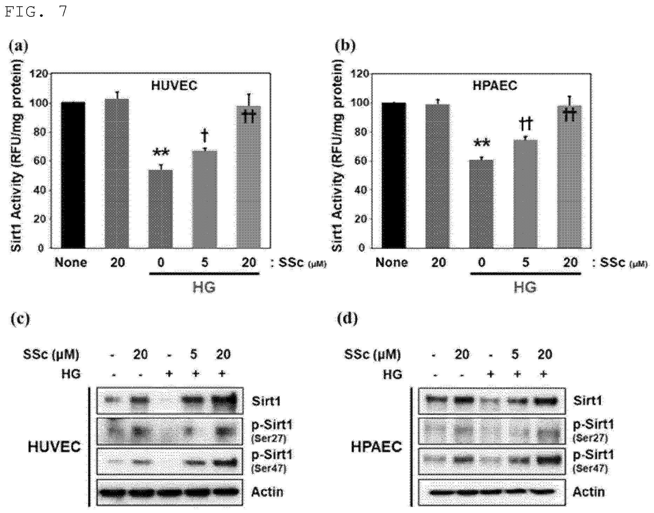

FIG. 7 shows the result of determination of whether or not Saikosaponin C alleviates the reduction of Sirt1 activity, protein expression, and phosphorylation in vascular endothelial cells (HUVEC, HPAEC) caused by high glucose as a high-fat diet. Sirt1 activity was measured using the Sirt1 Activity Assay Kit (FIG. 7A and FIG. 7B), and Sirt1 protein expression and phosphorylation (Ser27, Ser47) were measured through Western blotting (FIG. 7C and FIG. 7D).

The result shows that high glucose treatment induced a decrease in the enzyme activity, protein expression, and phosphorylation of Ser27/Ser47 residues of Sirt1 in HUVEC and HPAEC cells. However, pretreatment with Saikosaponin C significantly alleviated high glucose-induced decrease in Sirt1 enzyme activity, protein expression, and phosphorylation in HUVEC and HPAEC in a dose-dependent manner. Sirt1 protein is known to decrease in expression during the aging process and to exhibit a vascular anti-aging function. Therefore, these results support the anti-vascular aging effect of Saikosaponin C.

### Experimental Example 8. Analysis of underlying mechanisms by which Saikosaponin C exert anti-senescence activity against high glucose-induced vascular endothelial cell senescence (SA-β-gal staining, analysis of enzyme activity of NOS and Sirt1)

Similar to Experimental Example 1, a high-fat diet evaluation system at the cellular level was established.

Specifically, HUVEC cells, which are vascular endothelial cells, were pretreated with Saikosaponin C at a concentration of 20 µM for 2 hours, and then treated with high glucose at a concentration of 33 mM for 72 hours (for SA-β-gal staining) or 36 hours (for the analysis of enzyme activity of NOS and Sirt1). Then, SA-β-gal staining, NOS enzyme activity, and Sirt1 enzyme activity were analyzed. To analyze whether or not NOS and Sirt1 protein are involved in the anti-vascular aging effect of Saikosaponin C and the relationship between NOS and Sirt1 protein, L-N^{G}-nitro arginine methyl ester (L-NAME) (5 mM), an inhibitor of NOS, and EX-527 (10 µM), an inhibitor of Sirt1 protein, were used.

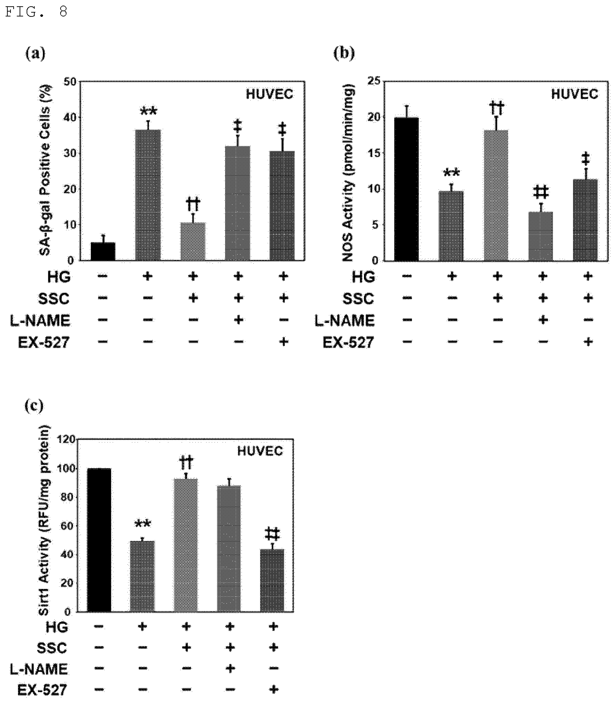

FIG. 8 shows the result of evaluation of the roles of NOS and Sirt1 protein in the anti-vascular aging effect of Saikosaponin C, as well as the relationship between these proteins, in order to evaluate the mechanism of action by which Saikosaponin C alleviates the aging of vascular endothelial cells (HUVECs) caused by high glucose corresponding to a high-fat diet. The senescence of vascular endothelial cells was assessed through intracellular SA-β-gal staining (FIG. 8A). The activities of NOS (FIG. 8B) and Sirt1 protein (FIG. 8C) were measured using the NOS Activity Assay Kit and the Sirt1 Activity Assay Kit, respectively.

As can be seen from FIG. 3, as expected, pretreatment with Saikosaponin C reduced high glucose-induced SA-β-gal positive HUVEC cells. However, in HUVEC cells pretreated with L-NAME, which is an inhibitor of NOS protein, or EX-527, which is an inhibitor of Sirt1 protein, the anti-vascular aging effect of Saikosaponin C was significantly abolished. In addition, both L-NAME pretreatment and EX-527 pretreatment abolished Saikosaponin C-mediated recovery of the reduction in NOS activity caused by high glucose, but L-NAME pretreatment did not abolish Saikosaponin C-mediated recovery of the reduction in Sirt1 activity caused by high glucose.

These results show that Sirt1 protein activity and subsequent NOS activity play an important role in the efficacy of Saikosaponin C in inhibiting vascular aging induced by high concentrations of glucose.

### Experimental Example 9. Inhibitory effect of Saikosaponin C on monocyte-endothelial cell adhesion caused by TNF-α in atherosclerosis (fluorescence analysis and protein expression analysis)

In Experimental Example 3, it was found that Saikosaponin C inhibits vascular endothelial cell senescence. Since arteriosclerosis is a representative disease caused by vascular aging, whether or not Saikosaponin C exhibits arteriosclerosis-inhibitory ability was determined. The first arteriosclerosis analysis was conducted through evaluation of monocyte-endothelial cell adhesion, which plays an important role in the early stage of arteriosclerosis.

Specifically, two types of vascular endothelial cells, namely, HUVEC (human umbilical vein endothelial cells) and HCAEC (human coronary artery endothelial cells), were pretreated with Saikosaponin C at concentrations of 5 µM and 20 µM for 2 hours, and then treated with TNF-α (20 ng/ml) for 8 hours. Then, adhesion of monocyte-vascular endothelial cell and expression of cell adhesion molecules (ICAM-1, VCAM-1) of vascular endothelial cells were observed. Human monocyte THP-1 cell line was used as a monocyte cell line and the THP-1 cells were labeled with Calcein AM (1 ug/ml per 1×10⁶ cells), a green fluorescent substance, for 30 minutes, and then spread on the two types of vascular endothelial cells. The adhesion of monocyte-endothelial cells was evaluated by imaging analysis using a confocal microscope and fluorescence analysis using a microplate reader, and expression of adhesion proteins (ICAM-1 and VCAM-1) was evaluated by Western blotting.

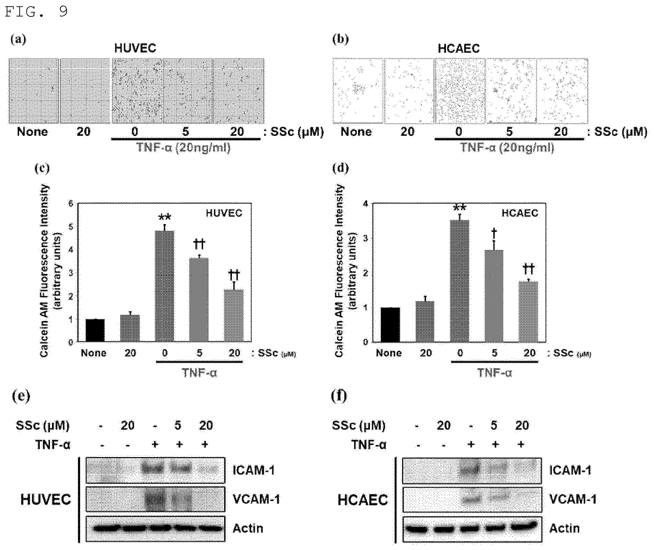

FIG. 9 shows the result of determination of whether or not Saikosaponin C inhibits monocyte-endothelial cell adhesion caused by TNF-α, an atherosclerotic agent. Calcein AM fluorescence was analyzed using a confocal microscope and a microplate reader, and the expression of cell adhesion proteins (ICAM-1, VCAM-1) were evaluated through Western blotting.

FIGS. 9A and 9B show the results of analysis of monocyte-endothelial cell adhesion in two types of vascular endothelial cells (HUVEC, HCAEC) using a confocal microscope, and FIGS. 9C and 9D show the results of analysis of Calcein AM fluorescence (at an excitation wavelength of 488 nm, and at an emission wavelength of 517 nm) in the same experiment using a microplate reader. FIGS. 9E and 9F show the results of Western blotting to determine the expression of adhesion proteins such as ICAM-1 and VCAM-1 in two types of vascular endothelial cells (HUVEC, HCAEC).

The result showed that monocyte-endothelial cell adhesion increased in both types of vascular endothelial cells (HUVEC, HCAEC) by TNF-α treatment and that this adhesion was inhibited when pretreated with Saikosaponin C. In addition, the result showed that Saikosaponin C inhibited the expression of adhesion proteins of vascular endothelial cells involved in monocyte-endothelial cell adhesion. Monocyte-endothelial cell adhesion is considered to be an initial step in the development of arteriosclerosis and thus adhesion proteins such as ICAM-1 and VCAM-1 are also vascular aging marker proteins. Therefore, these results mean that Saikosaponin C has anti-vascular aging efficacy and anti-atherosclerotic efficacy based thereon.

### Experimental Example 10. Analysis of effects of Saikosaponin C on inhibition of vascular smooth muscle cell migration induced by PDGF during atherosclerosis (wound healing and cell migration analysis)

As in Experimental Example 9 above, whether or not Saikosaponin C exhibits an arteriosclerosis-inhibitory effect was determined. The second arteriosclerosis analysis was conducted through evaluation of abnormal proliferation and migration of vascular smooth muscle cells, which are major mechanisms that indicate the full-scale progression of arteriosclerosis.

Specifically, two types of vascular smooth muscle cells, namely, AoSMC (human aortic smooth muscle cells) and CASMC (human coronary artery smooth muscle cells), were pretreated with Saikosaponin C at concentrations of 5 µM and 20 µM for 2 hours, and then treated with PDGF-BB (20 ng/ml), a platelet-derived growth factor (PDGF), for 24 hours. Then, the proliferation and migration of vascular smooth muscle cells were observed. A wound healing assay was performed to observe the proliferation and migration of vascular smooth muscle cells, and a transwell migration assay was performed to observe the migration ability of vascular smooth muscle cells.

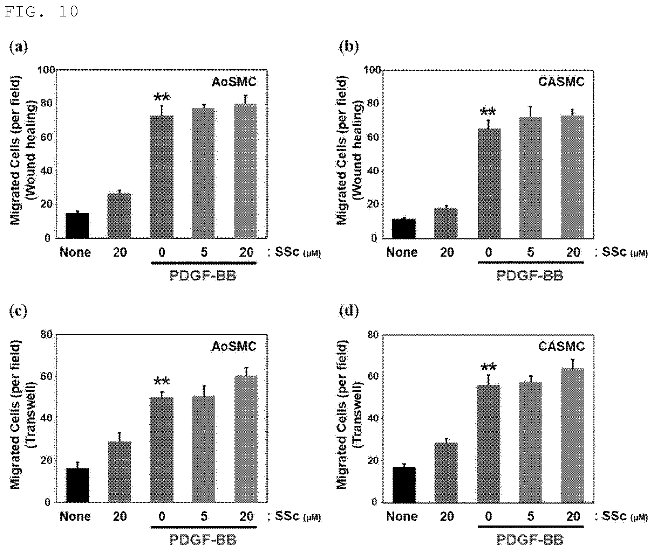

FIG. 10 shows the results of determination of whether or not Saikosaponin C inhibits abnormal proliferation and migration of vascular smooth muscle cells caused by PDGF-BB, which is involved in the progression of atherosclerosis. FIGS. 10A and 10B show the results of evaluating the proliferation and migration of vascular smooth muscle cells (AoSMC, CASMC) through a scratch wound healing assay. FIGS. 10C and 10D show the results of analyzing the cell migration ability of the same cells using a Transwell migration chamber with a pore size of 8.0 µm.

The result showed that the proliferation and migration of vascular smooth muscle cells increased in both types of vascular smooth muscle cells (AoSMC, CASMC) by PDGF-BB treatment. Unlike in Experimental Example 9, it was found that the increased proliferation and migration were not inhibited even when pretreated with Saikosaponin C. These results mean that the proliferation and migration of vascular smooth muscle cells are not involved in the anti-atherosclerotic effect of Saikosaponin C.

### Experimental Example 11. Analysis of effects of Saikosaponin C on macrophage foam cell formation in atherosclerosis (imaging analysis and absorbance analysis)

As in Experimental Examples 9 and 10 above, Saikosaponin C inhibited monocyte-endothelial cell adhesion caused by TNF-α, but did not inhibit abnormal proliferation and migration of vascular smooth muscle cells caused by PDGF. Therefore, another atherosclerosis phenotype analysis was conducted to determine whether or not Saikosaponin C exhibited an atherosclerosis-inhibitory effect. The third atherosclerosis analysis was conducted by observing macrophage-derived foam cell formation, which plays an important role in the early stage of atherosclerosis, along with monocyte-endothelial cell adhesion. Macrophage foam cell formation corresponds to the stage between monocyte-endothelial cell adhesion and abnormal proliferation and migration of vascular smooth muscle cells in the progression to atherosclerosis.

Specifically, THP-1 cells, a monocytic cell line, were differentiated into macrophages by treatment with PMA (phorbol 12-myristate 13-acetate) at a concentration of 150 nM for 48 hours. The differentiated macrophages were pretreated with Saikosaponin C at concentrations of 5 µM and 20 µM for 2 hours, and then treated with oxidized LDL (ox-LDL) (100 ug/ml), a foam cell inducer, for 24 hours. Then, the formation of foam cells from macrophages was observed. The foam cell formation was evaluated by microscopic analysis after Oil Red O staining of adsorbed lipid molecules and absorbance analysis using a microplate reader.

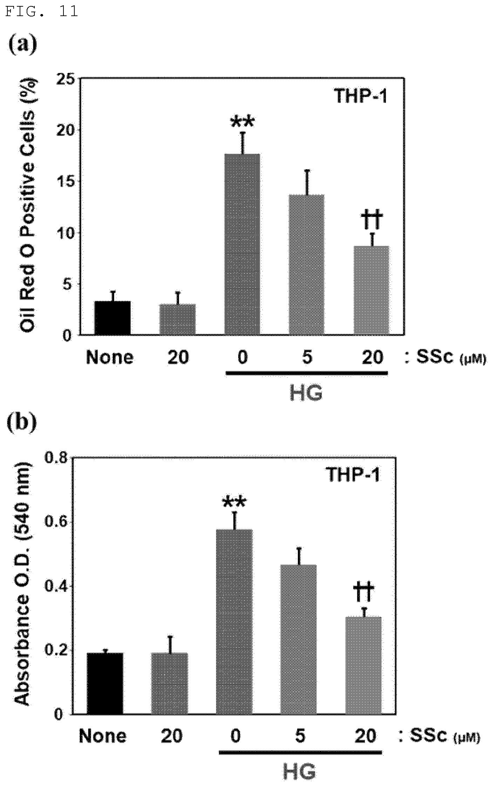

FIG. 11 shows the result of determination of whether or not Saikosaponin C inhibits the formation of macrophage foam cells by oxidized LDL (ox-LDL), an arteriosclerotic agent. FIG. 11 shows the results of analysis of the degree of macrophage foam cell formation after immobilizing cells with 10% formalin for 30 minutes and Oil Red O staining for 30 minutes. The nucleus was stained with hematoxylin dye for 1 minute. The degree of foam cell formation was analyzed by microscopically analyzing the degree of red coloring with Oil Red O dye as shown in FIG. 11A, or by measuring the absorbance at 540 nm with a microplate reader after eluting Oil Red O dye with 100% isopropanol for 10 minutes as shown in FIG. 11B.

The result showed that macrophages ingested a large amount of lipids and transformed into foam cells by oxidized LDL treatment and that foam cell formation by lipid deposition was suppressed when pretreated with Saikosaponin C. The conversion of macrophages into foam cells is considered a typical pathological finding of early atherosclerosis along with monocyte-endothelial cell adhesion. Therefore, these results mean that Saikosaponin C acts in the early stage of atherosclerosis and has an anti-atherosclerotic effect.

### Experimental Example 12. Inhibitory effect of Saikosaponin C on NLRP3 inflammasome activation in atherosclerosis (ELISA and protein expression analysis)

As in Experimental Examples 9, 10, and 11, whether or not Saikosaponin C exhibits an arteriosclerosis-inhibitory effect was determined. NLRP3 (NOD-like receptor protein-3), which acts as a sensor, forms a protein complex called "NLRP3 inflammasome" along with ASC, which acts as an adaptor, and caspase-1, which acts as an effector. NLRP3 inflammasome induces pyroptosis, a cell death related to inflammation, through the secretion of IL-1β, which is an inflammatory cytokine, and activation of caspase-1 in response to infection and cell damage. Above all, NLRP3 inflammasome is known to promote macrophage foam cell formation through IL-1β secretion and caspase-1 activation. The effect of Saikosaponin C of inhibiting macrophage foam cell formation by oxidized LDL in Experimental Example 11 above was demonstrated. Therefore, the fourth atherosclerosis analysis was conducted through the evaluation of NLRP3 inflammasome activity that promotes macrophage foam cell formation.

Specifically, THP-1 cells, a monocytic cell line, were differentiated into macrophages by treatment with PMA at a concentration of 150 nM for 48 hours. The differentiated macrophages were pretreated with Saikosaponin C at a concentration of 20 µM for 2 hours, and then treated with monosodium urate crystals (MSU), an NLRP3 inflammasome activator, at a concentration of 200 ug/ml for 6 hours. Then, IL-1β secretion and caspase-1 cleavage were analyzed. In addition, the cells were pretreated with MCC950 (1 µM), an NLRP3 inflammasome inhibitor, as a control for comparing the efficacy of Saikosaponin C, for 2 hours, similar to Saikosaponin C.

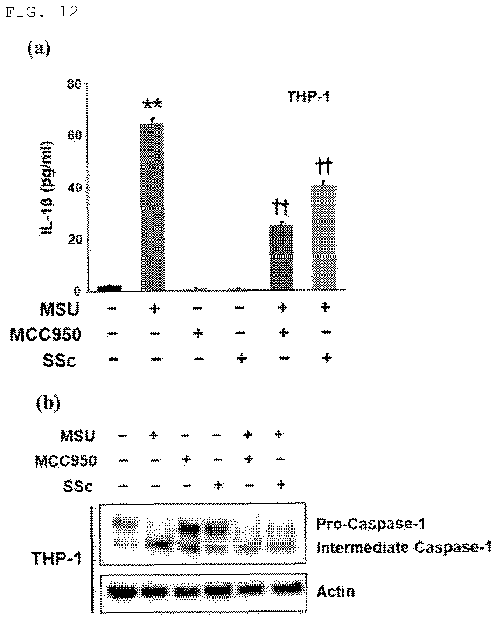

FIG. 12 shows the results of determination of whether or not Saikosaponin C inhibits the activity of NLRP3 inflammasome, a protein complex that promotes formation of macrophage foam cells. FIG. 12A shows the results of analysis of NLRP3 inflammasome activity by measurement of the secretion of IL-1β, an inflammatory cytokine, through Solid Phase Sandwich ELISA. FIG. 12B shows the results of analysis of NLRP3 inflammasome activity by evaluation of caspase-1 cleavage through Western blotting.

The result showed that IL-1β secretion and caspase-1 cleavage increased by treatment with monosodium urate monohydrate crystals (MSU) and that IL-1β secretion and caspase-1 cleavage were inhibited when pretreated with Saikosaponin C, similar to pretreatment with MCC950, an NLRP3 inflammasome inhibitor. Caspase-1 cleavage and subsequent IL-1β secretion are required for NLRP3 inflammasome activation. This effect of inhibiting IL-1β secretion and caspase-1 cleavage means that Saikosaponin C can inhibit NLRP3 inflammasome activity. Additionally, since the activated NLRP3 inflammasome promotes foam cell formation, these results once again demonstrate the anti-atherosclerotic efficacy of Saikosaponin C in the early stage of atherosclerosis through inhibition of macrophage foam cell formation.

In summary, Saikosaponin C inhibited vascular aging caused by vascular endothelial cell senescence and inhibited monocyte-endothelial cell adhesion and macrophage foam cell formation by the NLRP3 inflammasome, which play an important role in the early stage of atherosclerosis. Therefore, Saikosaponin C may be used as an atherosclerotic treatment based on inhibition of vascular aging. This new concept of arteriosclerosis treatment based on inhibition of vascular aging may be used singly for treatment of arteriosclerosis diseases and exhibit therapeutic effects, or may be used in combination with conventional therapeutic agents for arteriosclerosis to lower LDL-C and thus enhance therapeutic effects.

It will be apparent to those skilled in the art that various modifications and variations can be made in the disclosure without departing from the spirit or scope of the disclosure. Thus, it is intended that the disclosure cover such modifications and variations thereof, provided they fall within the scope of the appended claims and their equivalents.

## Claims

1. A pharmaceutical composition for treating or preventing a disease caused by vascular senescence comprising Saikosaponin C or a pharmaceutically acceptable salt thereof as an active ingredient.

2. The pharmaceutical composition according to claim 1, wherein the disease caused by vascular senescence is arteriosclerosis.

3. The pharmaceutical composition according to claim 1, wherein the vascular senescence is senescence of vascular endothelial cells.

4. The pharmaceutical composition according to claim 1, wherein the composition inhibits monocyte-endothelial cell adhesion, NLRP3 inflammasome activity, macrophage-derived foam cell formation, or macrophage-derived foam cell formation by NLRP3 inflammasome.

5. A food composition for ameliorating or preventing a disease caused by vascular aging comprising Saikosaponin C or a sitologically acceptable salt thereof as an active ingredient.

6. The food composition according to claim 5, wherein the disease caused by vascular aging is arteriosclerosis.

7. The food composition according to claim 5, wherein the vascular aging is aging of vascular endothelial cells.

8. The food composition according to claim 5, wherein the composition inhibits monocyte-endothelial cell adhesion, NLRP3 inflammasome activity, macrophage-derived foam cell formation, or macrophage-derived foam cell formation by NLRP3 inflammasome.

9. The food composition according to claim 5, wherein the food composition is a health functional food.

10. A pharmaceutical composition for treating or preventing a symptom caused by vascular senescence comprising Saikosaponin C or a pharmaceutically acceptable salt thereof as an active ingredient.

11. The pharmaceutical composition according to claim 10, wherein the symptom caused by vascular senescence is arteriosclerosis.

12. The pharmaceutical composition according to claim 10, wherein the symptom caused by vascular senescence comprises at least one selected from the group consisting of inhibition of vascular endothelial cell growth, increased vascular endothelial cell senescence, increased production of reactive oxygen species in vascular endothelial cells, decreased nitric oxide concentration in vascular endothelial cells, decreased expression or activity of NOS protein, decreased expression or activity of Sirt1 protein, increased monocyte-endothelial cell adhesion, increased NLRP3 inflammasome activity, increased formation of macrophage-derived foam cells, and increased formation of macrophage-derived foam cells by NLRP3 inflammasome.

13. A food composition for ameliorating or preventing a symptom caused by vascular aging comprising Saikosaponin C or a sitologically acceptable salt thereof as an active ingredient.

14. The food composition according to claim 13, wherein the symptom caused by vascular aging is arteriosclerosis.

15. The food composition according to claim 13, wherein the symptom caused by vascular aging comprises at least one selected from the group consisting of inhibition of vascular endothelial cell growth, increased vascular endothelial cell aging, increased production of reactive oxygen species in vascular endothelial cells, decreased nitric oxide concentration in vascular endothelial cells, decreased expression or activity of NOS protein, decreased expression or activity of Sirt1 protein, increased monocyte-endothelial cell adhesion, increased NLRP3 inflammasome activity, increased formation of macrophage-derived foam cells, and increased formation of macrophage-derived foam cells by NLRP3 inflammasome.

16. The food composition according to claim 13, wherein the food composition is a health functional food.
